# EUROPÄISCHE PATENTANMELDUNG

(11) **EP 1 974 668 A1**
(43) Veröffentlichungstag der Anmeldung: **01.10.2008**
(21) Anmeldenummer: 07022379.7
(22) Anmeldetag: 19.11.2007
(51) Int. Cl.: A61B 5/15

(54) **Stechsystem**

(30) Priorität: 29.03.2007 EP 07006519
(71) Anmelder: Roche Diagnostics GmbH, 68305 Mannheim (DE); F. Hoffmann-La Roche AG, 4070 Basel (CH)
(72) Erfinder: Harttig, Herbert, 67434 Neustadt (DE)
(74) Vertreter: Twelmeier Mommer & Partner

(57) **Zusammenfassung**

Stechsystem zum Gewinnen einer Körperflüssigkeitsprobe mittels einer von einem Stechantrieb (18) für eine Stichbewegung in Stichrichtung beschleunigte Lanzette (6), wobei das Stechsystem eine Einstelleinrichtung (12) zum Einstellen der Stichtiefe, mit der eine Lanzette (6) bei einem Einstich in den Körper eines Patienten eindringt, aufweist. Es ist vorgesehen, dass die Einstelleinrichtung (12) ein quer zur Stichrichtung bewegliches Anschlagelement (11) aufweist, das eine Unterseite (11b), die bei einem Stich dem Körper eines Patienten zugewandt ist, und eine Oberseite (11a) mit einer Anschlagfläche, gegen die bei einem Stich ein mit der Lanzette (6) verbundener Anschlag (10) anschlägt, aufweist, wobei das Anschlagelement (11) in Stichrichtung von der Anschlagfläche bis zur Unterseite (11b) eine sich quer zur Stichrichtung ändernde Ausdehnung hat, so dass durch Bewegen des Anschlagelements (11) quer zur Stichrichtung einstellbar ist, wie weit eine Lanzette (6) bei einem Stich mit ihrer Spitze über eine Unterseite (11b) des Anschlagelements (11) hervorragt. Beschrieben ist ferner ein Lanzettenmagazin für ein Stechsystem, wobei das Lanzettenmagazin mehrere Lanzetten (6) enthält und ein Anschlagelement (11) aufweist, das eine Unterseite (11b), die bestimmungsgemäß bei einem Stich dem Körper eines Patienten zugewandt ist, und eine Oberseite (11a) mit einer Anschlagfläche für einen bei einem Stich mit einer Lanzette (6) verbundenen Anschlag (10) aufweist, wobei die Anschlagfläche schräg zu der Unterseite (11b) verläuft.

## Beschreibung

Die Erfindung geht aus von einem Stechsystem zum Gewinnen einer Körperflüssigkeitsprobe mit den im Oberbegriff des Anspruchs 1 angegebenen Merkmalen.

Derartige Stechsysteme werden beispielsweise von Diabetikern verwendet, die mehrmals täglich ihren Blutzuckerspiegel überprüfen müssen und dafür eine Körperflüssigkeitsprobe, in der Regel Blut oder interstitielle Flüssigkeit, benötigen, die aus einer mit einem Stechsystem erzeugte Stichwunde gewonnen wird.

Für eine möglichst schmerzarme Probengewinnung sollte die Stichtiefe, mit der eine Lanzette bei einem Einstich in den Körper eines Patienten eindringt, nicht tiefer als nötig sein. Da die Dicken der oberen Hautschichten von Patient zu Patient verschieden sind und zudem von dem zur Probengewinnung gewählten Körperteil abhängen, weißen Stechsysteme üblicherweise eine Einstelleinrichtung zum Einstellen der Stichtiefe auf.

Aufgabe der Erfindung ist es einen Weg aufzuzeigen, wie bei einem Stechsystem kostengünstig eine zuverlässige Einstelleinrichtung zum Einstellen der Stichtiefe geschaffen werden kann.

Diese Aufgabe wird durch ein Stechsystem mit den im Anspruch 1 angegebenen Merkmalen gelöst. Vorteilhafte Weiterbildungen sind Gegenstand der Unteransprüche.

Bei einer erfindungsgemäßen Einstelleinrichtung wird die Stichtiefe mittels eines quer zur Stichrichtung beweglichen Anschlagelements eingestellt. Das Anschlagelement hat eine Unterseite, die bei einem Stich dem Körper eines Patienten zugewandt ist, und eine Oberseite mit einer Anschlagfläche, gegen die bei einem Stich ein mit der Lanzette verbundener Anschlag anschlägt.

Eine erfindungsgemäße Einstelleinrichtung kann mechanisch sehr einfach und deshalb zuverlässig und kostengünstig realisiert werden, beispielsweise durch ein keilförmiges Anschlagelement. Durch Verschieben des Anschlagelements quer zur Stichrichtung kann vorgegeben werden, auf welche Stelle der Anschlagfläche der mit der Lanzette verbundene Anschlag bei einem Stich trifft. Da das Anschlagelement in Stichrichtung von der Anschlagfläche bis zur Unterseite eine sich quer zur Stichrichtung ändernde Ausdehnung hat, lässt sich durch Querverschiebung des Anschlagelements festlegen, wie weit eine Lanzette bei einem Stich mit ihrer Spitze über die Unterseite des Anschlagelements hervorragt und somit die Stichtiefe vorgeben. Dabei ist es nicht erforderlich, dass das Anschlagelement exakt und nur quer zur Einstichrichtung verschoben werden kann. Es genügt, wenn das Anschlagelement zum Einstellen der Stichtiefe in einer Richtung bewegt wird, die auch eine Komponente quer zur Stichrichtung hat. Bevorzugt ist dabei, dass eine Bewegung des Anschlagelements zum Einstellen der Stichtiefe überwiegend quer zur Stichrichtung erfolgt.

Beispielsweise können die Anschlagfläche und die Unterseite ebene Flächen sein, die schräg zueinander verlaufen. Auf diese Weise ist eine Änderung der Stichtiefe proportional zu einer Verschiebung des Anschlagelements quer zur Stichrichtung. Möglich ist es aber auch die Anschlagfläche als eine gekrümmte Fläche auszubilden, so dass keine lineare Proportionalität zwischen einer Verschiebung des Anschlagelements quer zur Stichrichtung und einer Änderung der Stichtiefe besteht.

Bei einem erfindungsgemäßen Stechsystem ist es nicht erforderlich, dass das Körperteil, in dem eine Stichwunde erzeugt werden soll, bereits bei Auslösen des Stichs an der Unterseite des Anschlagelements anliegt. Möglich ist es nämlich beispielsweise auch, dass das Anschlagelement erst durch das Anschlagen des Anschlags der Lanzette gegen das Körperteil gedrückt wird. Dies kann beispielsweise dadurch erreicht werden, dass das Anschlagelement federnd an dem Stechgerät oder einem Lanzettenmagazin befestigt ist, so dass sich die Anschlagfläche beim Auftreffen des mit einer Lanzette verbundenen Anschlags in Stichrichtung federnd bewegen kann. Möglich ist es auch, das Anschlagelement starr anzuordnen, so dass eine Lanzette abgestoppt wird, sobald der mit ihr verbundene Anschlag an die Anschlagfläche anschlägt.

Der mit der Lanzette verbundene Anschlag, der mit dem Anschlagelement zur Begrenzung der Stichtiefe zusammenwirkt, kann beispielsweise als Schulter einer Lanzette ausgebildet sein. Insbesondere bei einer aus Blech ausgeschnittenen Flachlanzette lässt sich eine solche Schulter mit geringem Aufwand ausbilden. Möglich ist es auch den Anschlag mittels eines Lanzettenkörpers aus Kunststoff auszubilden, der ein Lanzettenspitze aus Metall, Keramik oder einem anderen ausreichend harten Material trägt. Es ist aber nicht erforderlich, dass der Anschlag wie bei den genannten Beispielen dauerhaft mit der Lanzette verbunden ist. Es genügt, wenn bei einem Stich der Anschlag mit der Lanzette verbunden ist, so dass ein Abstoppen des Anschlags auch ein Abstoppen der Lanzette bewirkt. Beispielsweise kann der Anschlag Teil des Lanzettenantriebs sein, der für einen Stich mit der Lanzette koppelt und diese anschließend wieder frei gibt.

Ein erfindungsgemäßes Stechsystem kann ein Stechgerät und austauschbare, in das Stechgerät einsetzbare Lanzettenmagazine, die jeweils mehrere Lanzettenmagazine enthalten, umfassen. Das Anschlagelement kann dabei Teil des Stechgeräts sein oder in einem derartigen Lanzettenmagazin enthalten sein.

Weitere Einzelheiten und Vorteile werden anhand eines Ausführungsbeispiels unter Bezugnahme auf die beigefügten Zeichnungen erläutert. Die dabei beschriebenen Merkmale können einzeln und in Kombination zum Gegenstand von Ansprüchen gemacht werden. Es zeigen:
- Figur 1: ein Ausführungsbeispiel eines Stechgeräts eines Stechsystems;
- Figur 2: eine Innenansicht zu Figur 1; und
- Figur 3: das Anschlagelement des dargestellten Stechsystems in einer Draufsicht.

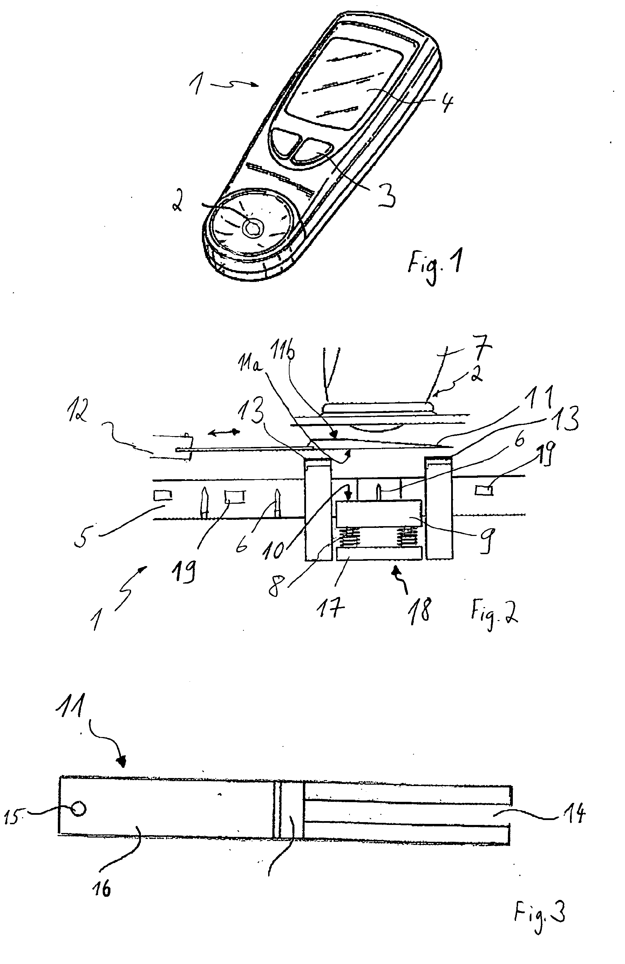

Figur 1 zeigt in einer schematischen Darstellung ein Ausführungsbeispiel eines Stechgeräts 1, das zusammen mit austauschbaren Lanzetten ein Stechsystem zum Gewinn einer Körperflüssigkeitsprobe bildet. Das Stechgerät 1 weist eine Geräteöffnung 2 auf, gegen die ein Körperteil zum Erzeugen einer Stichwunde gepresst wird. Das Stechgerät hat Bedienungselemente 3 in Form von Tasten und eine Anzeigeeinrichtung 4 in Form einer Flüssigkristallanzeige zum Anzeigen von Geräteeinstellungen und Untersuchungsergebnisse, die mit einer in das Stechgerät 1 integrierten Messeinrichtung an Körperflüssigkeitsproben gewonnen wurden. Das Stechgerät 1 hat ein Aufnahmefach (nicht dargestellt) für einen in Figur 2 dargestellten Lanzettenträger 5 mit mehreren Lanzetten 6. Das Aufnahmefach hat eine verschließbare Öffnung, die sich auf der Rückseite des in Figur 1 dargestellten Ausführungsbeispiels befindet.

Figur 2 zeigt schematisch die wesentlichen Komponenten des Stechsystems 1 in einer Innenansicht zu Figur 1 im Bereich der Gehäuseöffnung 2, an die ein Körperteil 7, üblicherweise ein Finger, zum Gewinnen einer Körperflüssigkeitsprobe angelegt wird. Zum Erzeugen einer Stichwunde wird von dem Stechantrieb 18 eine Lanzette 6 für eine Stichbewegung in Stichrichtung beschleunigt.

Bei dem dargestellten Ausführungsbeispiel sind die Lanzetten 6 auf einem bandförmigen Träger 5 angeordnet. Um die Lanzetten 6 nacheinander in eine Stechposition zu befördern, in der sie mit dem Stechantrieb 18 für eine Stichbewegung in der Stichrichtung beschleunigt werden können, ist ein nicht dargestellter Fortschaltmechanismus vorgesehen. Der Fortschaltmechanismus kann beispielsweise eine Wickeleinrichtung umfassen, mit welcher der bandförmiger Lanzettenträger 5, der beispielsweise aufgewickelt oder zickzackförmig zu einem Stapel gefaltet in das Aufnahmefach des Stechgeräts 1 eingesetzt sein kann, auf eine Rolle der Wickeleinrichtung aufgewickelt und auf diese Weise in Längsrichtung bewegt werden kann. Der bandförmige Lanzettenträger 5 kann insbesondere in einem austauschbaren Magazin enthalten sein, in dem er ähnlich wie das Band einer Tonbandkassette aufgewickelt ist. In einem solchen Magazin sind Abschnitte des Lanzettenträgers 5 mit unbenutzten Lanzetten 6 auf einer ersten Rolle aufgewickelt, wobei Abschnitte mit benutzten Lanzetten 6 auf eine von dem Fortschaltmechanismus angetriebene zweite Rolle aufgewickelt werden, so dass durch Betätigen des Fortschaltmechanismus der Träger 5 von der ersten Rolle abgewickelt und auf die zweite Rolle aufgewickelt und dabei eine frische Lanzette 6 in die Stechposition befördert werden kann.

Wie Figur 2 zeigt, sind die Lanzetten 6 auf dem bandförmigen Lanzettenträger 5 quer zu dessen Längsrichtung angeordnet. Zwischen zwei Lanzetten 6 befindet sich jeweils ein Testfeld 19 zur Untersuchung einer aus einer Stichwunde gewonnenen Körperflüssigkeitsprobe. Die Testfelder 19 können zur optischen, insbesondere photometrischen Bestimmung, einer Analytkonzentration Testchemikalien enthalten, die eine konzentrationsabhängige Änderung optischer Eigenschaften bewirken. Möglich ist es auch, die Testfelder 19 für eine elektrochemische oder physikalische Untersuchung einer Körperflüssigkeitsprobe einzurichten.

Der Stechantrieb 18 des dargestellten Ausführungsbeispiels koppelt mit dem Lanzettenträger 5, so dass dieser bei einem Stich zusammen mit einer in Stechposition positionierten Lanzette 6 in Stichrichtung bewegt wird. Der Stechantrieb 18 weist hierfür eine Aufnahme für den Lanzettenträger 5 auf, beispielsweise in Form einer Nut, durch die der Lanzettenträger 5 durchgeführt ist. Die Aufnahme 9 fasst den Lanzettenträger 5 bei einem Stich klemmend und gibt ihn nach einer erfolgten Einstich- und Rückführbewegung wieder frei. Auf diese Weise lässt sich erreichen, dass der Lanzettenträger 5 von dem Fortschaltmechanismus in Längsrichtung durch die Aufnahme 9 bewegt werden kann, eine Bewegung relativ zu der Aufnahme 9 in Stichrichtung jedoch verhindert wird.

Bei dem dargestellten Ausführungsbeispiel bildet die Aufnahme 9 des Stechantriebs 18 einen Anschlag 10 aus, der mit einer in Stechposition positionierten Lanzette 6 verbunden ist. Der von der Aufnahme 9 ausgebildete Anschlag wirkt zur Kontrolle der Stichtiefe mit dem Anschlagelement 11 einer Einstelleinrichtung 12 zum Einstellen der Stichtiefe, mit der eine Lanzette 6 bei einem Einstich in den Körper eines Patienten eindringt, zusammen. Die Aufnahme 9 ist über eine oder mehrere Ausgleichsfedern 8 auf einem Joch 17 gelagert, das bei einem Stich in Stichrichtung bewegt wird. Die Ausgleichsfedern gleichen bei einer kleinen Einstichtiefe den von dem Joch 17 vorgenommen Vorschub aus, so dass der Stechantrieb 18 nicht blockiert. Bei dem dargestellten Ausführungsbeispiel ist der Stechantrieb 18 bei einem Stich also über Ausgleichsfedern 8, die beim Abstoppen der Lanzette 6 komprimiert werden, an die Lanzette 6 gekoppelt. Der Stechantrieb 18 kann beispielsweise ein Rotorantrieb sein, bei dem ein Pleuel (nicht dargestellt) die Vorschubbewegung des Jochs 17 bewirkt. Derartige Rotorantriebe sind aus der US 2004/0092996 A1 bekannt.

Wird eine in der Stechposition positionierte Lanzette 6 zusammen mit dem Lanzettenträger 5 von dem Stechantrieb 18 in Stichrichtung bewegt, trifft zunächst der Lanzettenträger 5 mit einer der Geräteöffnung 2 zugewandten Längskante gegen eine Biegeeinrichtung 13, die bewirkt, dass sich der Lanzettenträger 5 in Längsrichtung biegt, so dass sich die Spitze der Lanzette 6 von dem Lanzettenträger 5 abhebt. Bei dem dargestellten Ausführungsbeispiel wird die Biegeeinrichtung 13 von zwei schräg zur Stichrichtung verlaufenden Schrägflächen gebildet, zwischen denen die Lanzette 6 für einen Stich in der Stechposition positioniert wird.

Der von der Aufnahme 9 des Stechantriebs 18 gebildete Anschlag 10 schlägt bei einer Stichbewegung an eine Oberseite 11a des in Figur 3 in einer Draufsicht dargestellten Anschlagelements 11 an. Das Anschlagelement 11 wird dabei von dem Stechantrieb 18 mit seiner Unterseite 11 b gegen ein an die Geräteöffnung 2 angelegtes Körperteil 7 gedrückt. Die Lanzette 6 kann deshalb bei einem Stich nur soweit in das Körperteil 7 eindringen, wie die Spitze der Lanzette 6 über die Unterseite 11 b hervorragt, wenn die Aufnahme 9 als Anschlag an der Oberseite 11a des Anschlagelements 11 anliegt.

Wie Figur 3 zeigt, hat das Anschlagelement 11 einen Schlitz 14, durch den sich eine Lanzette 6 bei einem Stich hindurchbewegt. Auf diese Weise kann der von der Aufnahme 9 des Stechantriebs 18 gebildete Anschlag 10 auf beiden Seiten des Schlitzes 14 von dem Anschlagelement 11 gestoppt werden, so dass Kippmomente vermieden werden können. Prinzipiell ist es jedoch ausreichend, wenn das Anschlagelement 11 nur auf einer Seite der Lanzette 6 mit dem Anschlag 10 in Wechselwirkung tritt, beispielsweise indem das Anschlagelement 11 eine Kante aufweist, an der die Lanzette 6 bei einem Stich vorbeigeführt wird und gegen die der mit der Lanzette 6 verbundene Anschlag 10 anschlägt.

Das Anschlagelement 11 ist quer zur Stichrichtung in Richtung des in Figur 2 dargestellten Doppelpfeils beweglich. Da das Anschlagelement 11 in Stichrichtung von der die Anschlagfläche ausbildenden Oberseite 11a bis zu der Unterseite 11 b eine Ausdehnung hat, die sich quer zur Stichrichtung ändert, lässt sich durch Bewegen des Anschlagelements 11 quer zur Stichrichtung einstellen, wie weit eine Lanzette 6 bei einem Stich mit ihrer Spitze über die Unterseite 11 b des Anschlagelements 11 hervorragt. Die Anschlagfläche 11a und die Unterseite 11 b des Anschlagelements 11 verlaufen schräg zu einander und sind jeweils eben.

Bei dem dargestellten Ausführungsbeispiel hat das Anschlagelement 11 einen keilförmigen Abschnitt, so dass sich die Dicke des Anschlagelements 11 in einer quer zur Stichrichtung verlaufenden Richtung ändert. Durch Verschieben des Anschlagelements 11 in dieser quer zur Stichrichtung verlaufenden Richtung lässt sich deshalb in einfacher Weise eine Einstellung der Stichtiefe erreichen. Das Anschlagelement 11 hat ein Kopplungselement 15 zum Ankoppeln an einen Verschiebemechanismus der Einstelleinrichtung 12. Bei dem dargestellten Ausführungsbeispiel ist das Kopplungselement 15 als Loch ausgebildet. Zum Ankoppeln an den Verschiebemechanismus kann ein Zapfen in das Loch 15 eingreifen und so eine formschlüssige Kopplung herstellen.

Das dargestellte Anschlagelement hat einen Fortsatz 16, der sich quer zur Stichrichtung erstreckt und an dem es gehalten ist. Der Fortsatz 16 schließt an den keilförmigen Abschnitt des Anschlagelements 11 an. Der Fortsatz 16 hat eine Dicke, die geringer als die durchschnittliche Dicke des die Anschlagfläche 11a ausbildenden Abschnitts, bei dem dargestellten Ausführungsbeispiel also des keilförmigen Abschnitts, und kann wegen des geringen Gewichts des Anschlagelements 11 auch als Folie ausgebildet sein. Auf diese Weise wird erreicht, dass das Anschlagelement 11 in Stichrichtung federnd beweglich ist. Bei einem Stich kann es deshalb von dem mit der Lanzette 6 verbundenen Anschlag gegen ein Körperteil 7 gedrückt werden. Sobald die Lanzette 6 von dem Stechantrieb 18 wieder zurückgezogen wird, federt das Anschlagelement 11 in seine ursprüngliche Lage zurück, so dass es vor einer Verschmutzung durch aus der erzeugten Stichwunde austretende Körperflüssigkeit geschützt ist. Bei dem dargestellten Ausführungsbeispiel liegt das Anschlagelement bei einem Einstich mit seiner Unterseite also nur zeitweilig am Körper des Patienten an. Möglich ist es aber auch, das Stechsystem derart auszubilden, dass das Anschlagelement 11 mit seiner Unterseite an dem Körperteil 7 eines Patienten anliegt, sobald dieses an die Geräteöffnung 2 angelegt wird.

Der Fortsatz 16 kann auch am dünnen Ende des die Anschlagfläche 11 a ausbildenden Abschnitts oder an beiden Enden angebracht werden. Mit der letztgenannten Ausführungsform lässt sich eine bessere Führung erreichen, die aber höhere Federkräfte mit sich bringt.

Bei dem dargestellten Ausführungsbeispiel hat der Fortsatz 16 eine Dicke von weniger 1 mm, insbesondere weniger als 0,5 mm, nämlich eine Dicke von 0,3 mm oder weniger. Der die Anschlagfläche 11a ausbildenden Abschnitt hat zwischen seinen Enden einen Dickenunterschied von mindestens 1 mm, insbesondere 1,5 mm, so dass die Stichtiefe in dem entsprechenden Bereich variiert werden kann. Bei dem dargestellten Ausführungsbeispiel variiert die Dicke des keilförmigen Abschnitts des Anschlagelements 11 zwischen 0 mm und etwa 1,5 mm bis 2 mm. Die Länge der Anschlagfläche 11a und damit die Länge des Schlitzes 14 kann mit mehr als 1 cm oder sogar mehr als 2 cm relativ groß gewählt werden, um den Einfluss von Positionierungsungenauigkeiten des Anschlagelements 11 auf die eingestellte Stechtiefe zu minimieren. Die Breite des Schlitzes 14 kann beispielsweise 2 mm bis 4 mm betragen.

### Bezugszahlen

- 1: Stechgerät
- 2: Geräteöffnung / Gehäuseöffnung
- 3: Bedienungselement
- 4: Anzeigeeinrichtung
- 5: Lanzettenträger
- 6: Lanzette
- 7: Körperteil
- 8: Feder
- 9: Aufnahme
- 10: Anschlag
- 11: Anschlagelement
- 11a: Oberseite / Anschlagfläche
- 11b: Unterseite
- 12: Einstellrichtung
- 13: Biegeeinrichtung
- 14: Schlitz
- 15: Kopplungselement
- 16: Fortsatz
- 17: Joch
- 18: Stechantrieb
- 19: Testfeld

## Patentansprüche

1. Stechsystem zum Gewinnen einer Körperflüssigkeitsprobe mittels einer von einem Stechantrieb (18) für eine Stichbewegung in Stichrichtung beschleunigte Lanzette (6), wobei das Stechsystem eine Einstelleinrichtung (12) zum Einstellen der Stichtiefe, mit der eine Lanzette (6) bei einem Einstich in den Körper eines Patienten eindringt, aufweist, **dadurch gekennzeichnet, dass**
die Einstelleinrichtung (12) ein quer zur Stichrichtung bewegliches Anschlagelement (11) aufweist, das eine Unterseite (11 b), die bei einem Stich dem Körper eines Patienten zugewandt ist, und eine Oberseite (11a) mit einer Anschlagfläche, gegen die bei einem Stich ein mit der Lanzette (6) verbundener Anschlag (10) anschlägt, aufweist,
wobei das Anschlagelement (11) in Stichrichtung von der Anschlagfläche bis zur Unterseite (11 b) eine sich quer zur Stichrichtung ändernde Ausdehnung hat, so dass durch Bewegen des Anschlagelements (11) quer zur Stichrichtung einstellbar ist, wie weit eine Lanzette (6) bei einem Stich mit ihrer Spitze über eine Unterseite (11 b) des Anschlagelements (11) hervorragt.

2. Stechsystem nach Anspruch 1, **dadurch gekennzeichnet, dass** die Anschlagfläche schräg zur Unterseite (11 b) verläuft.

3. Stechsystem nach einem der vorstehenden Ansprüche, **dadurch gekennzeichnet, dass** das Anschlagelement (11) einen keilförmigen Abschnitt hat.

4. Stechsystem nach einem der vorstehenden Ansprüche, **dadurch gekennzeichnet, dass** das Anschlagelement (11) einen sich quer zur Stichrichtung erstreckenden Fortsatz (16) aufweist, an dem es gehalten ist.

5. Stechsystem nach einem der vorstehenden Ansprüche, **dadurch gekennzeichnet, dass** das Anschlagelement (11) in Stichrichtung federnd beweglich ist.

6. Stechsystem nach einem Ansprüche 4 bis 5, **dadurch gekennzeichnet, dass** der Fortsatz (16) weniger als 1 mm, vorzugsweise weniger als 0,5 mm, dick ist.

7. Stechsystem nach einem der vorstehenden Ansprüche, **dadurch gekennzeichnet, dass** das Anschlagelement (11) bei einem Einstich mit seiner Unterseite (11 b) zumindest zeitweilig am Körper des Patienten anliegt.

8. Stechsystem nach einem der vorstehenden Ansprüche, **dadurch gekennzeichnet, dass** das der mit dem Anschlagelement (11) zusammenwirkende Anschlag Teil des Stechantriebs (18) ist.

9. Stechsystem nach einem der vorstehenden Ansprüche, **dadurch gekennzeichnet, dass** der Stechantrieb (18) bei einem Stich über eine Ausgleichsfeder (8), die bei einem Abstoppen der Lanzette (6) komprimiert wird, an eine Lanzette (6) gekoppelt ist.

10. Lanzettenmagazin für ein Stechsystem, wobei das Lanzettenmagazin mehrere Lanzetten (6) enthält, **dadurch gekennzeichnet, dass** das Lanzettenmagazin ein Anschlagelement (11) aufweist, das eine Unterseite (11b), die bestimmungsgemäß bei einem Stich dem Körper eines Patienten zugewandt ist, und eine Oberseite (11a) mit einer Anschlagfläche für einen bei einem Stich mit einer Lanzette (6) verbundenen Anschlag (10) aufweist, wobei die Anschlagfläche schräg zu der Unterseite (11 b) verläuft.
